# EUROPÄISCHE PATENTSCHRIFT

(11) **EP 3 282 984 B1**
(45) Veröffentlichungstag und Bekanntmachung des Hinweises auf die Patenterteilung: **22.04.2020**
(21) Anmeldenummer: 16715999.5
(22) Anmeldetag: 30.03.2016
(51) Int. Cl.: A61B 17/86

(54) **KNOCHENSCHRAUBE**
BONE SCREW
VIS À OS

(30) Priorität: 15.04.2015 AT 503012015
(43) Veröffentlichungstag der Anmeldung: 21.02.2018
(73) Patentinhaber: Pastl, Klaus, 4040 Lichtenberg (AT)
(72) Erfinder: Pastl, Klaus, 4040 Lichtenberg (AT)
(74) Vertreter: KLIMENT & HENHAPEL
(86) Internationale Anmeldenummer: PCT/AT2016/050084
(87) Internationale Veröffentlichungsnummer: WO 2016/164946

(56) Entgegenhaltungen:
- WO-A1-2013/164106

## Beschreibung

Die Erfindung betrifft einen Bolzen mit Außengewinde und einem zylindrischen Bolzenschaft aus allogenem, kortikalen Knochenmaterial für die chirurgisch operative Osteosynthese, wobei es sich bei dem Außengewinde um ein symmetrisches Spitz- oder Trapezgewinde handelt, das zumindest einen Gewindegang pro Millimeter aufweist, und das Knochenmaterial von Osteonen gebildet und mit Havers-Kanälen durchzogen ist, gemäß dem Oberbegriff von Anspruch 1.

Sofern sich das Außengewinde eines solchen Bolzens über die gesamte Längserstreckung des Bolzens erstreckt, kann auch von einem Gewindestift gesprochen werden. Ist der Bolzen ferner mit einem gewindelosen Kopf versehen, kann auch von einer Knochenschraube gesprochen werden. Im Folgenden wird vorzugsweise von Knochenschrauben gesprochen, wobei von dieser Bezeichnung auch Ausführungen in Form eines Gewindestifts umfasst sein sollen.

Knochenschrauben für die chirurgisch operative Osteosynthese werden in herkömmlicher Weise aus Metall bzw. Metalllegierungen gefertigt. Ferner sind Knochenschrauben aus resorbierbarem Material, etwa Polyglykolid und Polylaktid, bekannt, sowie Schrauben aus xenogenem Knochen. Knochenschrauben dieser Art weisen in der chirurgischen Praxis allerdings mehrere Nachteile auf. So müssen etwa Schrauben aus Metall bzw. Metalllegierungen einerseits durch eine Zweitoperation wieder entfernt werden, und unterliegen andererseits Veränderungen durch Korrosion. Damit erhöhen sich die Kosten im Gesundheitssystem. Außerdem besteht ein zusätzliches gesundheitliches Risiko für jeden Patienten durch eine neuerliche Operation, das bei Schrauben aus allogenem Knochen nicht besteht. Sämtliche resorbierbaren Materialien im menschlichen oder tierischen Körper wiederum bilden zwar je nach Material eine mehr oder weniger feste Brücke zwischen den zu osteosynthetisierenden Knochen, werden aber aufgelöst, was die Festigkeit der Osteosynthese der betroffenen Knochen negativ beeinflusst. Des Weiteren führen manche resorbierbare Osteosynthesematerialien während ihres Abbaus zu großen Osteolysen in dem umgebenden Knochen, also zu einem Wegweichen des Empfängerknochens von der Schraube. Xenogene (speziesfremde) Materialien wiederum führen zu Abstoßungsreaktionen und sind für die Osteosynthese auch deshalb ungeeignet, weil sie in den umgebenden Empfängerknochen nicht eingebaut, sondern abgestoßen und abgebaut werden, auch wenn das Eiweiß im Knochen vorher durch Hitze denaturiert wurde. Weiters trägt auch der unterschiedliche Elastizitätsmodul von boviner Corticalis und humaner Corticalis (Mensch ca. 16.000 N/mm², Rind ca. 22.000 bis 24.000 N/mm²) dazu bei, dass humanes Material wesentlich besser einheilen kann. Formfestigkeit und Elastizitätsmodul des corticalen Knochens sind dabei speziesabhängig.

Schrauben aus allogenem Knochen (Femur und Tibia-Corticalis) verfügen hingegen über mehrere Vorteile. Sie werden ohne Abstoßungsreaktion vaskularisiert und umgebaut, und sind vor allem für Osteosynthesen dort geeignet, wo kleine Knochenfragmente zusammengefügt werden müssen, da durch die Schraube bereits bei der Operation eine tragende Knochenbrücke entsteht, die sich vom Zeitpunkt der Operation an verbessert, indem sie sich umbaut und voll in den lebenden Knochen integriert und eingebaut wird. Im Gegensatz dazu stellen Metallschrauben eher ein Hindernis für die Knochenneubildung dar, insbesondere verringern sie durch deren bloße Präsenz die zur Verfügung stehende Oberfläche, die für die Knochenheilung vorhanden wäre. Abbaubare Materialien wiederum haben ihre maximale Festigkeit zum Zeitpunkt der Operation. Für sie gelten dieselben Nachteile wie für die Metallschrauben, des Weiteren nimmt die Festigkeit rapide ab, sobald der Abbauprozess eintritt, wodurch die zu osteosynthetisierende Knochenstelle zumindest vorübergehend wieder eine Schwächung erfährt.

Des Weiteren kann bei Knochenschrauben aus allogenem Knochen eine Zweitoperation zur Entfernung des Osteosynthesematerials entfallen, da der Knochen vollständig in eigenen Knochen umgewandelt (nicht resorbiert!) wird. Für den Patienten verringert sich somit das Operationsrisiko, für das Gesundheitssystem verringern sich gezwungenermaßen die Kosten. Schrauben aus allogenem Knochen stören auch nicht bei der Anwendung bildgebender Verfahren, im Gegensatz zu Metallschrauben, die störende Artefakte im MRI und CT hinterlassen. Auch Nachuntersuchungen sind problemlos möglich, und lassen eine bessere Beurteilung des Heilungserfolges zu.

Das Dokument WO2013/164106 A1 offenbart einen Bolzen gemäß dem Oberbegriff von Anspruch 1. Für die chirurgische Praxis geeignete Knochenschrauben wurden etwa in der EP 2384712 B1 des Anmelders beschrieben, der in weiterer Folge auch als nächstkommender Stand der Technik aufgefasst wird. Die darin beschriebenen Knochenschrauben weisen insbesondere einen mit einem Außengewinde versehenen Bolzenschaft auf, wobei es sich bei dem Außengewinde um ein symmetrisches Spitz- oder Trapezgewinde handelt, das zumindest einen Gewindegang pro Millimeter aufweist. Das symmetrische Spitz- oder Trapezgewinde verfügt dabei über den Vorteil, dass die der Einschraubrichtung zugewandte Flanke eines Gewindeganges denselben Winkel aufweist, wie seine abgewandte Flanke. Sie übt daher keinerlei Zugwirkung auf den Knochen aus. Im Zuge der operativen Osteosynthese wird stattdessen die Fixierung und Kompression der zu verbindenden Knochenteile zunächst durch entsprechende chirurgische Instrumente sichergestellt. In weiterer Folge wird eine Kernbohrung gesetzt, in das ein Gewinde geschnitten wird. Die Knochenschraube kann nun eingebracht werden, wobei die Knochenschraube die Knochenteile in einem stabilen Zustand mit vorgegebener Distanz hält, ohne dabei eine Kompression auszuüben, da ein symmetrisch ausgeführtes Spitz- oder Trapezgewinde kaum Zugbelastungen zulässt. Eine solche Knochenschraube stellt daher keine Zugschraube dar. Die Druckbelastung auf das Muttergewinde und auf das Schraubengewinde wird reduziert, bzw. auf Grund des symmetrischen Flankenwinkels zumindest auf beide Gewindeabschnitte gleich verteilt. Somit kann auf diese Art und Weise am besten Drucknekrosen des Knochens vorgebeugt bzw. entgegengewirkt werden. Aufgrund der hohen Gewindezahl von zumindest einem Gewindegang pro Millimeter und der damit verbundenen Reibungskräfte ist außerdem eine hohe Rotationsstabilität der Knochenschraube und vor allem eine große Oberfläche für die Knochenheilung gegeben.

Für eine Anwendbarkeit von Schrauben aus allogenem Knochen ist es aber insbesondere auch notwendig, sie hinsichtlich Eindrehwiderstand und Festigkeit zu optimieren. Knochenschrauben aus allogener, humaner Corticalis sind an sich sehr spröde und können bei Torsions- und Zugbelastungen leicht brechen. Belastungen der Knochenschraube treten aber nicht nur beim Eindrehen der Knochenschraube im Zuge der Operation auf, sondern auch in der postoperativen Heilungsphase. Da die gattungsgemäßen Knochenschrauben außerdem aus allogener, humaner Corticalis gewonnen werden, unterliegt das Ausgangsmaterial in seinen Eigenschaften Schwankungen, die sich auf die Qualität der aus diesem Ausgangsmaterial gefertigten Knochenschraube auswirken. Diese Schwankungen treten dabei nicht nur innerhalb ein- und desselben Knochens auf, sondern auch im Knochenmaterial unterschiedlicher Spender. Tatsächlich besteht hierin auch ein Grund dafür, dass Schrauben aus autologem oder allogenem Knochen bislang in der chirurgischen Praxis noch wenig Verbreitung gefunden haben.

Daher wurden im österreichischen Patent AT 511.943 des Anmelders Ausführungen für Schrauben aus allogenem, kortikalen Knochen vorgeschlagen, bei denen in einem Querschnitt normal zur Längsachse des Bolzenschaftes die Gesamtfläche der Havers-Kanäle höchstens 10% der Gesamtfläche des Querschnittes einnimmt. Ein Havers-Kanal ist der zentrale Knochenkanal in der Mitte eines Osteons, das das Grundbauelement der Kortikalis ist. Ein Osteon besteht dabei aus konzentrisch angeordneten Lamellen, die auch als Speziallamellen bezeichnet werden, einem zentral gelegenen Havers-Kanal und den, zwischen den Lamellen liegenden Osteozyten. Im Havers-Kanal verlaufen Blutgefäße, Bindegewebszellen und -fasern sowie einzelne Nervenfasern. Osteone und Havers-Kanäle kommen nur in der Kortikalis langer Röhrenknochen vor, wobei die Havers-Kanäle im Wesentlichen in Längsrichtung des Knochens verlaufen. Beim Zuschnitt von Knochenschrauben wird in herkömmlicher Weise der Symmetrie des Knochenaufbaus gefolgt und die Knochenschraube in Längsrichtung des Spenderknochens geschnitten, sodass die Längsachse des Bolzenschaftes im Wesentlichen in Längsrichtung des Spenderknochens verläuft. Somit verlaufen auch die Havers-Kanäle im Bolzenschaft im Wesentlichen in Längsrichtung des Bolzenschaftes.

Wie nun vom Anmelder festgestellt wurde, hängen die mechanischen Eigenschaften von Knochenschrauben aus allogenem, kortikalen Knochenmaterial zu einem beträchtlichen Teil von der verfügbaren Knochenmatrix ab. Eine flächenmäßige Zunahme der Havers-Kanäle im Querschnitt eines Knochens reduziert die verfügbare Knochenmatrix, wobei der flächenmäßige Anteil von Havers-Kanälen im Knochenquerschnitt nicht nur entlang der Längserstreckung des Knochens variiert, sondern auch zwischen dem Knochenmaterial unterschiedlicher Spender. Das im österreichischen Patent AT 511.943 vorgeschlagene Kriterium, dass in einem Querschnitt normal zur Längsachse des Bolzenschaftes die Gesamtfläche der Havers-Kanäle höchstens 10% der Gesamtfläche des Querschnittes einnehmen darf, stellt hierfür ein einfaches und leicht messbares Kriterium für die Auswahl des Ausgangsmaterials für die Herstellung von Knochenschrauben dar, das eine gute Qualität der Knochenschraube in verlässlicher Weise sicherstellt.

Leider erweist sich der Anteil von jenem Material, das sich bei Anwendung dieses Kriteriums als nicht verwendbar erweist, als hoch. Spenderknochen sind aber eine sehr begrenzte Ressource, mit der behutsam und mit bestmöglicher Verwertung umgegangen werden muss. Es wäre daher wünschenswert auch Material von weniger geeigneten Spenderknochen für die Herstellung von qualitativ hochwertigen Knochenschrauben verwenden zu können.

Mit anderen Worten besteht das Ziel der Erfindung in der Verwirklichung von Knochenschrauben erhöhter Festigkeit, sodass auch weniger geeignetes Spendermaterial für die Herstellung qualitativ hochwertiger Knochenschrauben verwendet werden kann.

Dieses Ziel wird durch die Merkmale von Anspruch 1 erreicht. Anspruch 1 bezieht sich auf einen Bolzen mit Außengewinde und einem zylindrischen Bolzenschaft aus allogenem, kortikalen Knochenmaterial für die chirurgisch operative Osteosynthese, wobei es sich bei dem Außengewinde um ein symmetrisches Spitz- oder Trapezgewinde handelt, das zumindest einen Gewindegang pro Millimeter aufweist, und das Knochenmaterial von Osteonen gebildet und mit Havers-Kanälen durchzogen ist. Erfindungsgemäß ist hierbei vorgesehen, dass die ein Gewindetal begrenzenden Gewindeflanken des symmetrischen Spitz- oder Trapezgewindes über einen Gewindetalabschnitt ineinander übergehen, dessen in einem Axialschnitt des Bolzens erscheinende Länge im Bereich von 0.02 mm bis 0.6 mm liegt, wobei der Gewindetalabschnitt von einem Kreisbogen mit einem Gewindetalradius von 0.01 mm bis 0.20 mm gebildet wird. Mithilfe eines erfindungsgemäß ausgeführten Gewindetalabschnittes kann die Bruchfestigkeit einer Knochenschraube um über 30% erhöht werden, wie Versuche des Anmelders gezeigt haben. Dieser überraschend starke Effekt kann nicht auf die bloße Vermeidung von Spannungsspitzen zurückgeführt werden, wie sie in der Mechanik bei der Auslegung von Bauteilen angestrebt wird. Der Effekt dieser deutlichen Erhöhung der Bruchfestigkeit bei Knochenschrauben mit Feingewinde wird vom Anmelder stattdessen auf die Struktur des verwendeten Knochenmaterials zurückgeführt. Die im erfindungsgemäß vorgesehenen Intervall genannten Längen des Gewindetalabschnittes liegen im Bereich der Abmessungen der im Knochenmaterial maßgeblichen Struktureinheiten wie etwa der Havers-Kanäle und der Osteone. Die das erfindungsgemäße Intervall definierenden Intervallgrenzen sind demnach auch vergleichbar mit den kleinsten auftretenden Durchmessern von Havers-Kanälen (etwa 20pm) und den größten Durchmessern von Osteonen (etwa 400µm). In anderen Worten könnte das erfindungsgemäß vorgesehene Intervall für die Längen der Gewindetalabschnitte auch so interpretiert werden, dass die ein Gewindetal begrenzenden Gewindeflanken des symmetrischen Spitz- oder Trapezgewindes über einen Gewindetalabschnitt ineinander übergehen, dessen in einem Axialschnitt des Bolzens erscheinende Länge oberhalb des durchschnittlichen Durchmessers der Havers-Kanäle des betreffenden Knochenmaterials liegt und unterhalb des durchschnittlichen Durchmessers der Osteone des Knochenmaterials. Die deutliche Erhöhung der Bruchfestigkeit wird vom Anmelder darauf zurückgeführt, dass die Gewindeflanken und der die Gewindeflanken verbindende Gewindetalabschnitt die Havers-Kanäle und vor allem die sie umgebenden Osteone in zunehmend flachem Winkel schneidet, je größer die Länge des Gewindetalabschnittes ist. Der erfindungsgemäß als Kreisbogen ausgeführte Gewindetalabschnitt weist einen Gewindetalradius von etwa 0.01 mm bis 0.20 mm auf, was 10 µm bis 200 µm entspricht. In diesem Bereich weist der entsprechende Kreisbogen eine Krümmung auf, die mit jener der Osteone vergleichbar ist, was den schleifenden Schnitt erklärt. Insbesondere im Fall kürzerer Gewindetalabschnitte wäre es aber auch denkbar den Kreisbogen durch einen linearen Abschnitt zu approximieren, sodass die beiden Gewindeflanken jeweils über eine Kante in den Gewindetalabschnitt übergehen. Vorzugsweise ist aber vorgesehen, dass der Übergang der Gewindeflanken in den Gewindetalabschnitt jeweils kantenlos erfolgt. Im Belastungsfall wird dabei jeweils der auf die Gewindeflanken und den Gewindetalabschnitt der Knochenschraube wirkende Druck auf das gesamte Gewebe des Knochenmaterials, also auf viele umliegende Osteone gleichmäßig aufgeteilt. Zug- und Druckbelastungen werden somit gleichmäßig und spannungsfrei übertragen und über eine größere Fläche auf das Knochenmaterial verteilt. Damit wird es möglich, dass auch minderwertigeres, poröseres Knochenmaterial für die Herstellung von Knochenschrauben mit gleichwertiger Bruchfestigkeit Verwendung finden kann.

In der Praxis haben sich konkret Gewindetalradien von 0.08 mm bis 0.20 mm, vorzugsweise 0.12 mm, bewährt.

Die Erfindung wird in weiterer Folge anhand der beiliegenden Zeichnungen näher erläutert. Es zeigen hierbei die
Fig. 1 eine schematische Darstellung einer Knochenschraube gemäß dem Stand der Technik,
Fig. 2 eine schematische Darstellung der Bruchstelle eines Knochens mit einem eingesetzten Bolzen gemäß der Erfindung im Rahmen einer operativen Osteosynthese,
Fig. 3 ein Detailausschnitt eines Querschnittes senkrecht zur Längsachse einer Knochenschraube zur Illustration des Aufbaus eines Osteons und seines Havers-Kanal sowie der relativen Lage eines Gewindetalabschnittes gemäß dem Stand der Technik (strichliert) und gemäß der Erfindung (durchgezogen),
Fig. 4 einen Ausschnitt eines Axialschnittes einer erfindungsgemäßen Bolzenschraube.

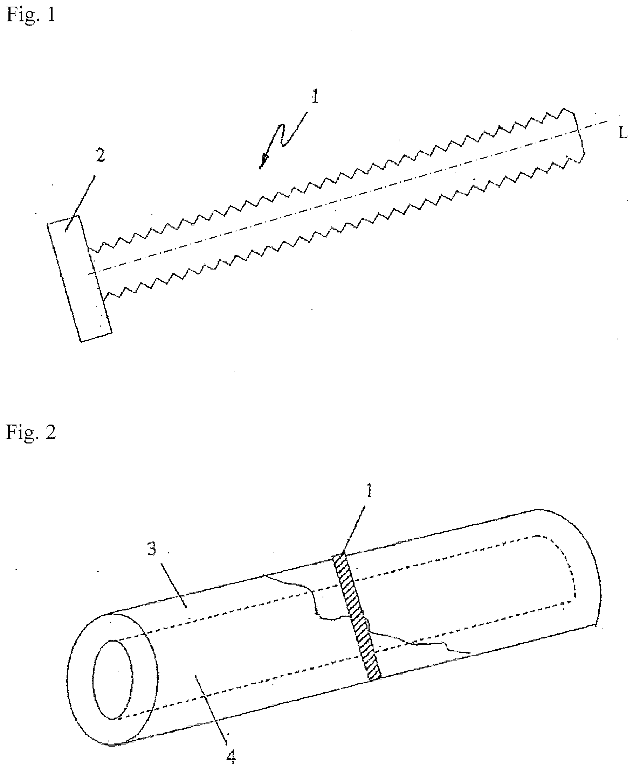

Die vorliegende Erfindung betrifft die Optimierung von Knochenschrauben 1 aus allogenem Knochen, wie sie schematisch in der Fig. 1 dargestellt ist, hinsichtlich ihrer Eigenschaften im Rahmen der operativen Osteosynthese, insbesondere in Bezug auf ihre Bruchfestigkeit.

Hierfür sind Knochenschrauben mit symmetrischem Spitz- oder Trapezgewinde vorgesehen, um keine Kompression auf die zu verbindenden Knochenteile auszuüben. Da außerdem die Festigkeit der Knochenverbindung im Wesentlichen von der Anzahl der Gewindegänge in der Kortikalis 3 des Empfängerknochens abhängt (siehe Fig. 2), und der Beitrag der Verschraubung entlang des spongiösen Knochens 4 nur gering ist, werden die Knochenschrauben so ausgeführt, dass sie eine möglichst hohe Anzahl an Gewindegängen pro Millimeter entlang des gesamten Bolzenschaftes aufweisen um sicherzustellen, dass die Kortikalis 3 in Verbindung mit einer entsprechend hohen Zahl an Gewindegängen steht. Bei einem Verhältnis von Gewindetiefe zu Gewindedurchmesser zwischen 0.10 und 0.15 ist hierzu etwa ein Produkt von dieser Verhältniszahl und der Anzahl der Gewindegänge pro Millimeter von 0.10 bis 0.30 vorgesehen. Das symmetrische Spitz- oder Trapezgewinde wird außerdem über die gesamte Länge des Bolzenschaftes mit konstantem Gewindedurchmesser geführt, sodass sich eine Knochenschraube 1 wie in der Fig. 1 dargestellt ergibt. Die Knochenschraube 1 selbst kann einen Vierkant, Sechskant oder Sternkopf als Schraubenkopf 2 aufweisen (siehe Fig. 1), der aber lediglich dem Einbringen von Drehmoment im Zuge des Einschraubens dient, und nach Platzierung der Knochenschraube 1 abgeschnitten wird, sodass eine Konfiguration gemäß Fig. 2 erreicht wird.

Im Zuge der operativen Osteosynthese wird zunächst die Fixierung und Kompression der zu verbindenden Knochenteile durch entsprechende chirurgische Instrumente sichergestellt. In weiterer Folge wird eine Kernbohrung gesetzt, in das ein Gewinde geschnitten wird. Die erfindungsgemäße Knochenschraube 1 kann nun etwa mit einem Steckschraubenschlüssel mit Drehmoment eingebracht werden, wobei die Knochenschraube 1 die Knochenteile entlang des spongiösen Knochens 4 und der Kortikalis 3 quert, und in einem stabilen Zustand mit vorgegebener Distanz hält, ohne dabei eine Kompression auszuüben, da ein symmetrisch ausgeführtes Spitz- oder Trapezgewinde kaum Zugbelastungen zulässt (siehe Fig. 2). Die dargestellte Knochenschraube 1 stellt daher keine Zugschraube dar. Aufgrund der hohen Gewindezahl und der damit verbundenen Reibungskräfte ist aber dennoch hohe Rotationsstabilität der Knochenschraube 1 gegeben.

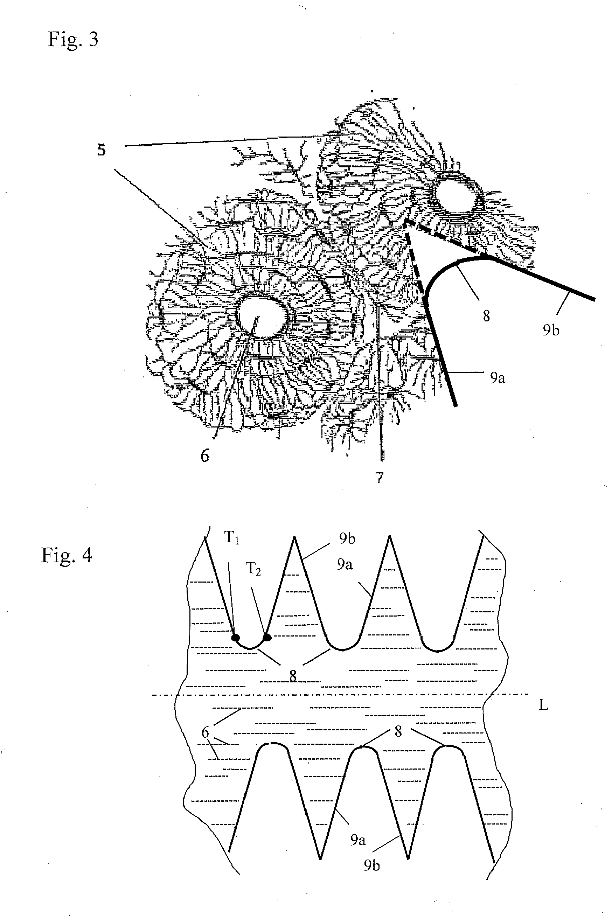

Zur Erläuterung der erfindungsgemäßen Merkmale wird nun zunächst auf die Fig. 3 Bezug genommen. Die Fig. 3 zeigt einen Detailausschnitt eines Querschnittes senkrecht zur Längsachse einer Knochenschraube 1 zur Illustration des Aufbaus eines Osteons 5 und seines Havers-Kanals 6 sowie der relativen Lage eines Gewindetalabschnittes 8 gemäß dem Stand der Technik (strichliert) und gemäß der Erfindung (durchgezogen). Da es sich bei der Fig. 3 um einen Schnitt normal zur Längsachse L der Knochenschraube 1 handelt und nicht um einen Axialschnitt, ist zwar der Gewindetalabschnitt ersichtlich, aber kein Gewindetalradius ablesbar. Ein Osteon 5 erscheint in diesem Schnitt als konzentrisch angeordnete Lamellen, die auch als Speziallamellen bezeichnet werden, dem zentral gelegenen Havers-Kanal 6 und den zwischen den Lamellen liegenden Osteozyten. Im Havers-Kanal 6 verlaufen Blutgefäße, Bindegewebszellen und -fasern sowie einzelne Nervenfasern. Die einzelnen Osteone 5 sind wiederum durch sogenannte Schaltlamellen 7 begrenzt und miteinander verbunden, wobei die Havers-Kanäle 6 der einzelnen Osteone 5 durch Querkanäle untereinander verbunden sind, die ebenfalls Blutgefäße enthalten und auch als Volkmann-Kanäle bezeichnet werden (in der Fig. 3 nicht ersichtlich).

Osteone 5 und Havers-Kanäle 6 kommen nur in der Kortikalis 3 langer Röhrenknochen vor, wobei die Havers-Kanäle 6 im Wesentlichen in Längsrichtung des Spenderknochens verlaufen. Beim Zuschnitt von Knochenschrauben 1 wird in herkömmlicher Weise der Symmetrie des Knochenaufbaus gefolgt und die Knochenschraube 1 in Längsrichtung des Spenderknochens geschnitten, sodass die Längsachse L des Bolzenschaftes im Wesentlichen in Längsrichtung des Spenderknochens verläuft. Somit verlaufen auch die Havers-Kanäle 6 im Bolzenschaft der Knochenschraube 1 im Wesentlichen parallel zur Längsachse L des Bolzenschaftes, wie anhand der Fig. 4 angedeutet ist.

Die Fig. 4 zeigt einen Ausschnitt einer erfindungsgemäßen Knochenschraube 1 aus allogenem, kortikalen Knochenmaterial im Axialschnitt, also in Längsrichtung des zylindrischen Bolzenschaftes und die Längsachse L enthaltend. In der Fig. 4 sind ferner die Achsen der innerhalb des Knochenmaterials verlaufenden Havers-Kanäle 6 eingezeichnet. Die Fig. 4 ist dabei schematisch zu verstehen und nicht maßstabsgetreu. Sie zeigt ferner ein als Spitzgewinde ausgeführtes Außengewinde, das einen zylindrischen Bolzenschaft spiralförmig umläuft. Das symmetrische Spitzgewinde weist zumindest einen Gewindegang pro Millimeter auf, wobei jeder Gewindegang zwei Gewindeflanken 9a, 9b aufweist, die über einen Gewindetalabschnitt 8 ineinander übergehen. Im gezeigten Ausführungsbeispiel ist der Übergang kantenlos ausgeführt, sodass die den Gewindetalabschnitt 8 begrenzenden Gewindeflanken 9a, 9b Tangenten des anschließenden Gewindetalabschnittes 8 darstellen. Die Länge des Gewindetalabschnittes 8 ist im Axialschnitt gemäß der Fig. 4 durch die Bogenlänge des Gewindetalabschnittes 8 zwischen den Tangentialpunkten T₁, T₂ der jeweiligen Gewindeflanke 9a, 9b ablesbar. Erfindungsgemäß liegt diese im Axialschnitt des Bolzens ablesbare Länge des Gewindetalabschnittes 8 im Bereich von 0.02 mm bis 0.6 mm. Falls der Übergang von den Gewindeflanken 9a, 9b zum Gewindetalabschnitt 8 jeweils durch eine Kante gebildet ist, wird die Länge des Gewindetalabschnittes 8 im Axialschnitt durch die Bogen- oder Streckenlänge des Gewindetalabschnittes 8 zwischen den Kanten ablesbar.

Die im erfindungsgemäß vorgesehenen Intervall genannten Längen des Gewindetalabschnittes 8 liegen im Bereich der Abmessungen der im Knochenmaterial maßgeblichen Struktureinheiten wie etwa der Havers-Kanäle 6 und der Osteone 5, wie in der Fig. 3 schematisch illustriert ist. Die deutliche Erhöhung der Bruchfestigkeit wird wie bereits erwähnt darauf zurückgeführt, dass die Gewindeflanken 9a, 9b und der die Gewindeflanken 9a, 9b verbindende Gewindetalabschnitt 8 die Havers-Kanäle 6 und vor allem die sie umgebenden Osteone 5 in zunehmend flachem Winkel schneiden, je größer die Länge des Gewindetalabschnittes 8 ist. Das erfindungsgemäß vorgesehene Intervall kann im Fall eines als Kreisbogen ausgeführten Gewindetalabschnittes 8 in Gewindetalradien von etwa 0.01 mm bis 0.20 mm umgerechnet werden, was 10 µm bis 200 µm entspricht. Diese Kreisbögen und der ihnen zugeordnete Gewindetalradius kann einem Axialschnitt gemäß der Fig. 4 entnommen werden, wobei wie bereits festgehalten die Fig. 4 bloß schematisch und nicht maßstabsgetreu ausgeführt ist. Im genannten Intervall der Gewindetalradien weist der entsprechende Kreisbogen jedenfalls eine Krümmung auf, die mit jener der Osteone 5 vergleichbar ist, wie auch der Fig. 3 entnommen werden kann, was den schleifenden Schnitt erklärt. Im Belastungsfall wird somit jeweils der auf die Gewindeflanken 9a, 9b und den Gewindetalabschnitt 8 der Knochenschraube 1 wirkende Druck auf das gesamte Gewebe des Knochenmaterials, also auf viele umliegende Osteone 5 gleichmäßig aufgeteilt. Zug- und Druckbelastungen werden somit gleichmäßig und spannungsfrei übertragen und über eine größere Fläche auf das Knochenmaterial verteilt. Auf diese Weise erklärt sich die überaus starke Zunahme der Bruchfestigkeit der Knochenschraube 1 von über 30%. Damit wird es möglich, dass auch minderwertigeres, poröseres Knochenmaterial für die Herstellung von Knochenschrauben 1 mit gleichwertiger Bruchfestigkeit Verwendung finden kann.

Somit gelingt es mithilfe der Erfindung Knochenschrauben 1 mit erhöhter Festigkeit herzustellen, sodass auch weniger geeignetes Spendermaterial für die Herstellung qualitativ hochwertiger Knochenschrauben 1 verwendet werden kann.

## Patentansprüche

1. Bolzen mit Außengewinde und einem zylindrischen Bolzenschaft aus allogenem, kortikalen Knochenmaterial für die chirurgisch operative Osteosynthese, wobei es sich bei dem Außengewinde um ein symmetrisches Spitz- oder Trapezgewinde handelt, das zumindest einen Gewindegang pro Millimeter aufweist, und das Knochenmaterial von Osteonen (5) gebildet und mit Havers-Kanälen (6) durchzogen ist, wobei die ein Gewindetal begrenzenden Gewindeflanken (9a, 9b) des symmetrischen Spitz- oder Trapezgewindes über einen Gewindetalabschnitt (8) ineinander übergehen, dessen in einem Axialschnitt des Bolzens erscheinende Länge im Bereich von 0.02 mm bis 0.6 mm liegt, **dadurch gekennzeichnet, dass**
der Gewindetalabschnitt (8) von einem Kreisbogen mit einem Gewindetalradius von 0.01 mm bis 0.20 mm gebildet wird.

2. Bolzen mit Außengewinde nach Anspruch 1, **dadurch gekennzeichnet, dass** der Übergang der Gewindeflanken (9a, 9b) in den Gewindetalabschnitt (8) jeweils kantenlos erfolgt.

3. Bolzen mit Außengewinde nach Anspruch 1 oder 2, **dadurch gekennzeichnet, dass** der Gewindetalabschnitt (8) von einem Kreisbogen mit einem Gewindetalradius von 0.08 mm bis 0.20 mm, vorzugsweise 0.12 mm, gebildet wird.

## Claims

1. A bolt with an external thread and a cylindrical bolt shank of allogeneic, cortical bone material for surgically operative osteosynthesis, wherein the external thread is a symmetrical angular or trapezoidal thread, which has at least one thread turn per millimeter, and the bone material is formed by osteons (5) and is permeated by Haversian canals (6), wherein the thread flanks (9a, 9b), which delimit a thread valley, of the symmetrical angular or trapezoidal thread converge into each other via a thread valley portion (8), whose length, in an axial section of the bolt, lies in the range of 0.02 mm to 0.6 mm, **characterized in that** the thread valley portion (8) is formed by a circular arc with a thread valley radius of 0.01 mm to 0.20 mm.

2. A bolt with an external thread according to claim 1, **characterized in that** the transition of the thread flanks (9a, 9b) into the thread valley portion (8) takes place in each case without edges.

3. A bolt with an external thread according to claim 1 or 2, **characterized in that** the thread valley portion (8) is formed by a circular arc with a thread valley radius of 0.08 mm to 0.20 mm, preferably 0.12 mm.

## Revendications

1. Boulon avec un filetage extérieur et une tige de boulon cylindrique faite de matériau osseux cortical allogène pour l'ostéosynthèse chirurgicale, dans lequel le filetage extérieur est un filet triangulaire ou trapézoïdal symétrique qui présente au moins une spire de filet par millimètre, et le matériau osseux est formé d'ostéons (5) et parcouru par des canaux de Havers (6), dans lequel les flancs de filet (9a, 9b) délimitant un fond de filet du filetage triangulaire ou trapézoïdal symétrique se fondent l'un dans l'autre par l'intermédiaire d'une partie de fond de filet (8) dont la longueur apparaissant dans une coupe axiale du boulon est comprise entre 0,02 mm et 0,6 mm, **caractérisé en ce que** la partie de fond de filet (8) est formée par un arc de cercle ayant un rayon de fond de filet de 0,01 mm à 0,20 mm.

2. Boulon avec un filetage extérieur selon la revendication 1, **caractérisé en ce que** la transition de chacun des flancs de filet (9a, 9b) dans la partie de fond de filet (8) se fait sans arête.

3. Boulon avec un filetage extérieur selon la revendication 1 ou 2, **caractérisé en ce que** la partie de fond de filet (8) est formée par un arc de cercle ayant un rayon de fond de filet de 0,08 mm à 0,20 mm, de préférence de 0,12 mm.
